# EUROPÄISCHE PATENTSCHRIFT

(11) **EP 3 149 485 B1**
(45) Veröffentlichungstag und Bekanntmachung des Hinweises auf die Patenterteilung: **03.03.2021**
(21) Anmeldenummer: 15723725.6
(22) Anmeldetag: 22.05.2015
(51) Int. Cl.: G01N 33/68

(54) **VERFAHREN ZUR DIAGNOSE EINER DURCH DEN ALTERNATIVEN WEG DES KOMPLEMENTSYSTEMS VERMITTELTEN KRANKHEIT ODER EINES RISIKOS HIERFÜR**
METHOD FOR DIAGNOSING A DISEASE OR A RISK TO DEVELOP A DISEASE TRANSMITTED VIA THE ALTERNATIVE PATHWAY OF THE COMPLEMENT SYSTEM
PROCÉDÉ DE DIAGNOSTIC D'UNE MALADIE MÉDIÉE PAR LA VOIE ALTERNE DU SYSTÈME DU COMPLÉMENT OU D'UN RISQUE POUR CELLE-CI

(30) Priorität: 26.05.2014 DE 102014107380
(43) Veröffentlichungstag der Anmeldung: 05.04.2017
(73) Patentinhaber: Eberhard Karls Universität Tübingen Medizinische Fakultät, 72074 Tübingen (DE)
(72) Erfinder: DAMMEIER, Sascha, 72070 Tuebingen (DE); KOERTVÉLY, Eloed, 72135 Dettenhausen (DE); UEFFING, Marius, 72076 Tübingen (DE)
(74) Vertreter: Witte, Weller & Partner Patentanwälte mbB
(86) Internationale Anmeldenummer: PCT/EP2015/061437
(87) Internationale Veröffentlichungsnummer: WO 2015/181088

(56) Entgegenhaltungen:
- EP-A1- 0 287 509
- WO-A1-2012/170556
- WO-A1-2013/071702
- US-A1- 2003 092 620
- US-A1- 2013 040 851
- YASUO YAMAUCHI ET AL: "RECOMBINANT AND NATIVE ZYMOGEN FORMS OF HUMAN COMPLEMENT FACTOR D", THE JOURNAL OF IMMUNOLOGY, THE AMERICAN ASSOCIATION OF IMMUNOLOGISTS, US, Bd. 152, 1. Januar 1994 (1994-01-01), Seiten 3645-3652, XP002933645, ISSN: 0022-1767
- HYE YEONG MIN ET AL: "Adipsin, the adipocyte serine protease: gene structure and control of expression by tumor necrosis factor", NUCLEIC ACIDS RESEARCH, Bd. 14, Nr. 22, 1. Januar 1986 (1986-01-01), Seiten 8879-8892, XP055198361, ISSN: 0305-1048, DOI: 10.1093/nar/14.22.8879 & DATABASE UniProt [Online] 23. Oktober 1986 (1986-10-23), XP007923159, Database accession no. P03953
- WANG T ET AL: "Elevation of urinary adipsin in preeclampsia: correlation with urine protein concentration and the potential use for a rapid diagnostic test", HYPERTENS, LIPPINCOTT WILLIAMS & WILKINS, US, vol. 64, no. 4, 1 October 2014 (2014-10-01), pages 846-851, XP009183720, ISSN: 0194-911X, DOI: 10.1161/HYPERTENSIONAHA.113.02688
- Douwe H. Biesma ET AL: "A family with complement factor D deficiency", JOURNAL OF CLINICAL INVESTIGATION, vol. 108, no. 2, 15 July 2001 (2001-07-15) , pages 233-240, XP055426230, US ISSN: 0021-9738, DOI: 10.1172/JCI200112023

SEQUENZPROTOKOLL
<110> Eberhard Karls Universität Tübingen Medizinische Fakultät
<120> Verfahren zur Diagnose einer durch den alternativen Weg des Komplementsystems vermittelten Krankheit oder eines Risikos hierfür
<130> 5402P527
<160> 13
<170> PatentIn Version 3.5
<210> 1
<211> 228
<212> PRT
<213> Homo sapiens
<400> 1
   Ile Leu Gly Gly Arg Glu Ala Glu Ala His Ala Arg Pro Tyr Met Ala
   1               5                   10                  15
   Ser Val Gln Leu Asn Gly Ala His Leu Cys Gly Gly Val Leu Val Ala
               20                  25                  30
   Glu Gln Trp Val Leu Ser Ala Ala His Cys Leu Glu Asp Ala Ala Asp
           35                  40                  45
   Gly Lys Val Gln Val Leu Leu Gly Ala His Ser Leu Ser Gln Pro Glu
       50                  55                  60
   Pro Ser Lys Arg Leu Tyr Asp Val Leu Arg Ala Val Pro His Pro Asp
   65                  70                  75                  80
   Ser Gln Pro Asp Thr Ile Asp His Asp Leu Leu Leu Leu Gln Leu Ser
                   85                  90                  95
   Glu Lys Ala Thr Leu Gly Pro Ala Val Arg Pro Leu Pro Trp Gln Arg
               100                 105                 110
   Val Asp Arg Asp Val Ala Pro Gly Thr Leu Cys Asp Val Ala Gly Trp
           115                 120                 125
   Gly Ile Val Asn His Ala Gly Arg Arg Pro Asp Ser Leu Gln His Val
       130                 135                 140
   Leu Leu Pro Val Leu Asp Arg Ala Thr Cys Asn Arg Arg Thr His His
   145                 150                 155                 160
   Asp Gly Ala Ile Thr Glu Arg Leu Met Cys Ala Glu Ser Asn Arg Arg
                   165                 170                 175
   Asp Ser Cys Lys Gly Asp Ser Gly Gly Pro Leu Val Cys Gly Gly Val
               180                 185                 190
   Leu Glu Gly Val Val Thr Ser Gly Ser Arg Val Cys Gly Asn Arg Lys
           195                 200                 205
   Lys Pro Gly Ile Tyr Thr Arg Val Ala Ser Tyr Ala Ala Trp Ile Asp
       210                 215                 220
   Ser Val Leu Ala
   225
<210> 2
<211> 233
<212> PRT
<213> Homo sapiens
<400> 2
   Pro Pro Arg Gly Arg Ile Leu Gly Gly Arg Glu Ala Glu Ala His Ala
   1               5                   10                  15
   Arg Pro Tyr Met Ala Ser Val Gln Leu Asn Gly Ala His Leu Cys Gly
               20                  25                  30
   Gly Val Leu Val Ala Glu Gln Trp Val Leu Ser Ala Ala His Cys Leu
           35                  40                  45
   Glu Asp Ala Ala Asp Gly Lys Val Gln Val Leu Leu Gly Ala His Ser
       50                  55                  60
   Leu Ser Gln Pro Glu Pro Ser Lys Arg Leu Tyr Asp Val Leu Arg Ala
   65                  70                  75                  80
   Val Pro His Pro Asp Ser Gln Pro Asp Thr Ile Asp His Asp Leu Leu
                   85                  90                  95
   Leu Leu Gln Leu Ser Glu Lys Ala Thr Leu Gly Pro Ala Val Arg Pro
               100                 105                 110
   Leu Pro Trp Gln Arg Val Asp Arg Asp Val Ala Pro Gly Thr Leu Cys
           115                 120                 125
   Asp Val Ala Gly Trp Gly Ile Val Asn His Ala Gly Arg Arg Pro Asp
       130                 135                 140
   Ser Leu Gln His Val Leu Leu Pro Val Leu Asp Arg Ala Thr Cys Asn
   145                 150                 155                 160
   Arg Arg Thr His His Asp Gly Ala Ile Thr Glu Arg Leu Met Cys Ala
                   165                 170                 175
   Glu Ser Asn Arg Arg Asp Ser Cys Lys Gly Asp Ser Gly Gly Pro Leu
               180                 185                 190
   Val Cys Gly Gly Val Leu Glu Gly Val Val Thr Ser Gly Ser Arg Val
           195                 200                 205
   Cys Gly Asn Arg Lys Lys Pro Gly Ile Tyr Thr Arg Val Ala Ser Tyr
       210                 215                 220
   Ala Ala Trp Ile Asp Ser Val Leu Ala
   225                 230
<210> 3
<211> 232
<212> PRT
<213> Homo sapiens
<400> 3
   Pro Arg Gly Arg Ile Leu Gly Gly Arg Glu Ala Glu Ala His Ala Arg
   1               5                   10                  15
   Pro Tyr Met Ala Ser Val Gln Leu Asn Gly Ala His Leu Cys Gly Gly
               20                  25                  30
   Val Leu Val Ala Glu Gln Trp Val Leu Ser Ala Ala His Cys Leu Glu
           35                  40                  45
   Asp Ala Ala Asp Gly Lys Val Gln Val Leu Leu Gly Ala His Ser Leu
       50                  55                  60
   Ser Gln Pro Glu Pro Ser Lys Arg Leu Tyr Asp Val Leu Arg Ala Val
   65                  70                  75                  80
   Pro His Pro Asp Ser Gln Pro Asp Thr Ile Asp His Asp Leu Leu Leu
                   85                  90                  95
   Leu Gln Leu Ser Glu Lys Ala Thr Leu Gly Pro Ala Val Arg Pro Leu
               100                 105                 110
   Pro Trp Gln Arg Val Asp Arg Asp Val Ala Pro Gly Thr Leu Cys Asp
           115                 120                 125
   Val Ala Gly Trp Gly Ile Val Asn His Ala Gly Arg Arg Pro Asp Ser
       130                 135                 140
   Leu Gln His Val Leu Leu Pro Val Leu Asp Arg Ala Thr Cys Asn Arg
   145                 150                 155                 160
   Arg Thr His His Asp Gly Ala Ile Thr Glu Arg Leu Met Cys Ala Glu
                   165                 170                 175
   Ser Asn Arg Arg Asp Ser Cys Lys Gly Asp Ser Gly Gly Pro Leu Val
               180                 185                 190
   Cys Gly Gly Val Leu Glu Gly Val Val Thr Ser Gly Ser Arg Val Cys
           195                 200                 205
   Gly Asn Arg Lys Lys Pro Gly Ile Tyr Thr Arg Val Ala Ser Tyr Ala
       210                 215                 220
   Ala Trp Ile Asp Ser Val Leu Ala
   225                 230
<210> 4
<211> 231
<212> PRT
<213> Homo sapiens
<400> 4
   Arg Gly Arg Ile Leu Gly Gly Arg Glu Ala Glu Ala His Ala Arg Pro
   1               5                   10                  15
   Tyr Met Ala Ser Val Gln Leu Asn Gly Ala His Leu Cys Gly Gly Val
               20                  25                  30
   Leu Val Ala Glu Gln Trp Val Leu Ser Ala Ala His Cys Leu Glu Asp
           35                  40                  45
   Ala Ala Asp Gly Lys Val Gln Val Leu Leu Gly Ala His Ser Leu Ser
       50                  55                  60
   Gln Pro Glu Pro Ser Lys Arg Leu Tyr Asp Val Leu Arg Ala Val Pro
   65                  70                  75                  80
   His Pro Asp Ser Gln Pro Asp Thr Ile Asp His Asp Leu Leu Leu Leu
                   85                  90                  95
   Gln Leu Ser Glu Lys Ala Thr Leu Gly Pro Ala Val Arg Pro Leu Pro
               100                 105                 110
   Trp Gln Arg Val Asp Arg Asp Val Ala Pro Gly Thr Leu Cys Asp Val
           115                 120                 125
   Ala Gly Trp Gly Ile Val Asn His Ala Gly Arg Arg Pro Asp Ser Leu
       130                 135                 140
   Gln His Val Leu Leu Pro Val Leu Asp Arg Ala Thr Cys Asn Arg Arg
   145                 150                 155                 160
   Thr His His Asp Gly Ala Ile Thr Glu Arg Leu Met Cys Ala Glu Ser
                   165                 170                 175
   Asn Arg Arg Asp Ser Cys Lys Gly Asp Ser Gly Gly Pro Leu Val Cys
               180                 185                 190
   Gly Gly Val Leu Glu Gly Val Val Thr Ser Gly Ser Arg Val Cys Gly
           195                 200                 205
   Asn Arg Lys Lys Pro Gly Ile Tyr Thr Arg Val Ala Ser Tyr Ala Ala
       210                 215                 220
   Trp Ile Asp Ser Val Leu Ala
   225                 230
<210> 5
<211> 230
<212> PRT
<213> Homo sapiens
<400> 5
   Gly Arg Ile Leu Gly Gly Arg Glu Ala Glu Ala His Ala Arg Pro Tyr
   1               5                   10                  15
   Met Ala Ser Val Gln Leu Asn Gly Ala His Leu Cys Gly Gly Val Leu
               20                  25                  30
   Val Ala Glu Gln Trp Val Leu Ser Ala Ala His Cys Leu Glu Asp Ala
           35                  40                  45
   Ala Asp Gly Lys Val Gln Val Leu Leu Gly Ala His Ser Leu Ser Gln
       50                  55                  60
   Pro Glu Pro Ser Lys Arg Leu Tyr Asp Val Leu Arg Ala Val Pro His
   65                  70                  75                  80
   Pro Asp Ser Gln Pro Asp Thr Ile Asp His Asp Leu Leu Leu Leu Gln
                   85                  90                  95
   Leu Ser Glu Lys Ala Thr Leu Gly Pro Ala Val Arg Pro Leu Pro Trp
               100                 105                 110
   Gln Arg Val Asp Arg Asp Val Ala Pro Gly Thr Leu Cys Asp Val Ala
           115                 120                 125
   Gly Trp Gly Ile Val Asn His Ala Gly Arg Arg Pro Asp Ser Leu Gln
       130                 135                 140
   His Val Leu Leu Pro Val Leu Asp Arg Ala Thr Cys Asn Arg Arg Thr
   145                 150                 155                 160
   His His Asp Gly Ala Ile Thr Glu Arg Leu Met Cys Ala Glu Ser Asn
                   165                 170                 175
   Arg Arg Asp Ser Cys Lys Gly Asp Ser Gly Gly Pro Leu Val Cys Gly
               180                 185                 190
   Gly Val Leu Glu Gly Val Val Thr Ser Gly Ser Arg Val Cys Gly Asn
           195                 200                 205
   Arg Lys Lys Pro Gly Ile Tyr Thr Arg Val Ala Ser Tyr Ala Ala Trp
       210                 215                 220
   Ile Asp Ser Val Leu Ala
   225                 230
<210> 6
<211> 229
<212> PRT
<213> Homo sapiens
<400> 6
   Arg Ile Leu Gly Gly Arg Glu Ala Glu Ala His Ala Arg Pro Tyr Met
   1               5                   10                  15
   Ala Ser Val Gln Leu Asn Gly Ala His Leu Cys Gly Gly Val Leu Val
               20                  25                  30
   Ala Glu Gln Trp Val Leu Ser Ala Ala His Cys Leu Glu Asp Ala Ala
           35                  40                  45
   Asp Gly Lys Val Gln Val Leu Leu Gly Ala His Ser Leu Ser Gln Pro
       50                  55                  60
   Glu Pro Ser Lys Arg Leu Tyr Asp Val Leu Arg Ala Val Pro His Pro
   65                  70                  75                  80
   Asp Ser Gln Pro Asp Thr Ile Asp His Asp Leu Leu Leu Leu Gln Leu
                   85                  90                  95
   Ser Glu Lys Ala Thr Leu Gly Pro Ala Val Arg Pro Leu Pro Trp Gln
               100                 105                 110
   Arg Val Asp Arg Asp Val Ala Pro Gly Thr Leu Cys Asp Val Ala Gly
           115                 120                 125
   Trp Gly Ile Val Asn His Ala Gly Arg Arg Pro Asp Ser Leu Gln His
       130                 135                 140
   Val Leu Leu Pro Val Leu Asp Arg Ala Thr Cys Asn Arg Arg Thr His
   145                 150                 155                 160
   His Asp Gly Ala Ile Thr Glu Arg Leu Met Cys Ala Glu Ser Asn Arg
                   165                 170                 175
   Arg Asp Ser Cys Lys Gly Asp Ser Gly Gly Pro Leu Val Cys Gly Gly
               180                 185                 190
   Val Leu Glu Gly Val Val Thr Ser Gly Ser Arg Val Cys Gly Asn Arg
           195                 200                 205
   Lys Lys Pro Gly Ile Tyr Thr Arg Val Ala Ser Tyr Ala Ala Trp Ile
       210                 215                 220
   Asp Ser Val Leu Ala
   225
<210> 7
<211> 10
<212> PRT
<213> Kuenstliche Sequenz
<220>
<223> Sonstige Angaben
<400> 7
   Pro Pro Arg Gly Arg Ile Leu Gly Gly Arg
   1               5                   10
<210> 8
<211> 9
<212> PRT
<213> Kuenstliche Sequenz
<220>
<223> Sonstige Angaben
<400> 8
   Pro Arg Gly Arg Ile Leu Gly Gly Arg
   1               5
<210> 9
<211> 8
<212> PRT
<213> Kuenstliche Sequenz
<220>
<223> Sonstige Angaben
<400> 9
   Arg Gly Arg Ile Leu Gly Gly Arg
   1 5
<210> 10
<211> 7
<212> PRT
<213> Knstliche Sequenz
<220>
<223> Sonstige Angaben
<400> 10
   Gly Arg Ile Leu Gly Gly Arg
   1 5
<210> 11 <211> 6
<212> PRT
<213> Kuenstliche Sequenz
<220>
<223> Sonstige Angaben
<400> 11
   Arg Ile Leu Gly Gly Arg
   1 5
<210> 12
<211> 5
<212> PRT
<213> Kuenstliche Sequenz
<220>
<223> Sonstige Angaben
<400> 12
   Ile Leu Gly Gly Arg
   1 5
<210> 13
<211> 30
<212> PRT
<213> Kuenstliche Sequenz
<220>
<223> Sonstige Angaben
<400> 13
   Met His Ser Trp Glu Arg Leu Ala Val Leu Val Leu Leu Gly Ala Ala
   1 5 10 15
   Ala Cys Ala Ala Pro Pro Arg Gly Arg Ile Leu Gly Gly Arg
   20 25 30

## Beschreibung

Die vorliegende Erfindung betrifft ein Verfahren zur Diagnose einer Krankheit oder eines Risikos für die Entwicklung einer Krankheit, insbesondere einer solchen, die durch den alternativen Weg des Komplementsystems vermittelt wird. Sie betrifft ferner die Verwendung eines neuen diagnostischen Markers.

Das Komplementsystem ist ein System von Plasmaproteinen, das im Zuge der Immunantwort aktiviert werden kann. Es ist Teil des angeborenen Immunsystems. Das menschliche Komplementsystem besteht aus mehr als 30 Proteinen, die im Blutplasma gelöst oder zellgebunden sind. Sie dienen der Abwehr von Mikroorganismen, haben jedoch auch stark zellzerstörende Eigenschaften und können, wenn sie unreguliert wirken, im Laufe vieler Krankheiten für Gewebeschäden verantwortlich sein.

Man unterscheidet drei Wege, durch die das Komplementsystem aktiviert wird, nämlich den meist über Antikörper induzierten klassischen Weg, den über das Mannose bindende Lektin aktivierten Lektinweg, und den spontanen und Antikörperunabhängigen alternativen Weg.

Ein geschwindigkeitsbestimmendes Enzym des alternativen Wegs ist der sogenannte Komplementfaktor D (CFD; EC 3.4.21.46), der auch als Adipsin oder C3-Proactivator-Konvertase, bezeichnet wird. CFD ist eine Serinprotease, die vorwiegend im Fettgewebe synthetisiert und in den Blutkreislauf sezerniert wird. CFD lässt sich darüber hinaus in verschiedenen Geweben finden. CFD spaltet den Komplementfaktor B. Es ist beschrieben, dass in Säugetierzellen das Enzym als inaktives Proenzym (proCFD) oder Zymogen synthetisiert wird und durch Abspaltung von 5 N-terminalen Aminosäuren in die reife und aktive Form, d.h. das CFD, überführt wird.

Im Stand der Technik wird CFD als Biomarker bei krankhaft fettleibigen Patienten oder Tieren beschrieben: Bienertová-Vaškù et al. (2011), Adipsin (Complement Factor D) as a new biomarker of body fat distribution in extremely obese central-European population, in "Body Fat: Composition, Measurements and Reduction Procedures", New York, Novapublishers, S. 14-27; Searfoss et al. (2003), Adipsin, a biomarker of gastrointestinal toxicity mediated by a functional gamma-secretase inhibitor, J. Biol. Chem., Vol. 278(46), S. 46107-46116; Miner et al. (1993), Adipsin expression and growth in rats as influenced by insulin and somatotropin, Physiol. Behav. Vol. 54(2), S. 207-212.

CFD wird ferner als ein Risikomarker in Bezug auf eine koronare Herzerkrankung beschrieben: Prentice et al. (2013), Proteomic risk markers for coronary heart disease and stroke: validation and mediation of randomized trial hormone therapy effects on these diseases, Genome Med. Vol. 5(12):112.

CFD wird mit einer Vielzahl weiterer Erkrankungen in Verbindung gebracht, wie bspw. beschrieben in den folgenden Patentdokumenten: WO 2012/170556, WO 2012/146778, WO 2011/136080, WO 2010/075519, WO 2008/137236, WO 2009/017405, RU 2232991, US 2003/092620, WO 2013/071702, WO 2013/071703, WO 2007/141004, WO 2007/030919, EP 0287509. Die mit CFD in Verbindung stehenden Erkrankungen lassen ich auch über die Datenbank "MalaCards" des Weizmann Institute of Science identifizieren (Sucheingabe "CFD").

In jüngster Zeit sind solche Krankheiten in den Mittelpunkt des Interesses gerückt, die über den alternativen Weg des Komplementsystems vermittelt werden. Diese sind zusammengefasst in Holers (2008), The Spectrum of Complement Alternative Pathway-Mediated Diseases, Immunological Reviews, Vol. 223, Seiten 300 bis 316.

Lo et al. (2014), Adipsin is an adipokine that improves better cell function in diabetes, Cell, 158(1): 41-53, beschreiben einen Zusammenhang zwischen dem alternativen Weg des Komplementsystems und der Entstehung von Diabetes mellitus Typ 2. In einer Subgruppe von untersuchten Patienten wurden erniedrigte Spiegel von CFD nachgewiesen, die mit einem erhöhten Risiko für einen Funktionsausfall der β-Zellen korrelierten. Die Autoren äußern den Wunsch nach einem Verfahren, mit dem Patienten hinsichtlich ihres Risikos einer Erkrankung klassifiziert werden können.

Spong et al. (2006), Deficient alternative complement pathway activation due to factor D deficiency by 2 novel mutations in the complement factor D gene in a family with meningococcal infections, Blood, 107: 4865-4870, beschreibt zwei Punktmutationen in CFD, die in Patienten festgestellt wurden, welche von einer Meningitis betroffen sind. Durch die Punktmutationen sei die Aktivierung des alternativen Wegs des Komplementsystems beeinträchtigt.

In der US 2003/0092620 werden Verfahren zur Behandlung von verschiedensten Patienten beschrieben. Es ist erwähnt, dass die bedürftigen Patienten identifiziert werden können, indem anhand von klinischen Proben eine Genotypisierung von CFD auf Single-Nucleotid-Polymorphismen (SNPs) erfolgt oder der mRNA-Spiegel bestimmt wird.

Die im Stand der Technik verfügbaren Verfahren haben sich jedoch in der Praxis nicht bewährt.

Vor diesem Hintergrund liegt der vorliegende Erfindung die Aufgabe zugrunde, ein Verfahren zur Diagnose einer Krankheit oder eines Risikos für die Entwicklung einer Krankheit, insbesondere einer solchen bereitzustellen, die durch den alternativen Weg des Komplementsystems vermittelt wird.

Diese Aufgabe wird gelöst durch ein Ex-vivo-Verfahren zur Diagnose einer Krankheit oder eines Risikos für die Entwicklung ei-ner Krankheit bei einem Lebewesen, das folgende Schritte aufweist:
(1) Untersuchung einer von einem Lebewesen bereitgestellten biologischen Probe auf das Vorhandensein von zumindest einer Isoform des Komplementfaktors D (isoCFD), die im Vergleich zu CFD an ihrem N-Terminus um 1 bis 4 Aminosäuren verlängert ist, und/oder im Vergleich zu proCFD an ihrem N-Terminus um 1 bis 4 Aminosäuren verkürzt ist (isoCFD),
(2) Stellen einer positiven Diagnose bei einem positiven Nachweis von zumindest einer isoCFD in der biologischen Probe, oder Stellen einer negativen Diagnose bei einem negativen Nachweis von zumindest einer isoCFD in der biologischen Probe.

Die Erfinder haben überraschenderweise erkannt, dass sich in einer biologischen Probe eines Lebewesens, das erkrankt ist oder einem Risiko für die Entwicklung einer Krankheit unterliegt, bislang weitgehend unbekannte Isoformen des Komplementfaktors D (CFD) finden lassen, die in einem gesunden Referenzlebewesen nicht bzw. nur unterhalb der Nachweisgrenze vorhanden sind.

Diese Erkenntnis war überraschend. So gibt es zwar Vorschläge im Stand der Technik, CFD oder proCFD als biologischen bzw. diagnostischen Marker heranzuziehen, nicht jedoch Isoformen von CFD.

Isoformen menschlichen CFD sind weitgehend unbekannt.

Yamauchi et al. (1994), Recombinant and native zymogen forms of human complement factor D, Journal of Immunology, Vol. 152, Seiten 3645 bis 3653, beschreiben ein Isoenzym von CFD, das im Urin eines Patienten mit Fanconi-Syndrom gefunden wurde. Die Autoren haben den Urin der Patienten lediglich als Quelle großer Mengen von CFD verwendet, da bekannt ist, dass sie von einer Proteinurie betroffen sind und deshalb besonders hohe Proteinmengen über den Urin ausscheiden. Die Autoren haben die diagnostische Relevanz der aufgefundenen Isoform von CFD jedoch nicht erkannt.

Erfindungsgemäß wird unter einer "Isoform des Komplementfaktors D" bzw. "isoCFD" ein Abkömmling von CFD verstanden, der sich gegenüber der reifen bzw. aktiven Form von CFD, nachfolgend als "CFD" bezeichnet, und der unreifen Form von CFD (proCFD) unterscheidet. Nach einer bevorzugten Ausfiihrungsvariante weist isoCFD einen anderen isoelektrischen Punkt (pl) als CFD und proCFD auf. Nach einer weiteren bevorzugten Ausführungsvariante weist die isoCFD ein anderes Molekulargewicht als CFD und proCFD auf. Nach einer noch weiteren bevorzugten Ausführungsvariante weist die isoCFD in ihrer Aminosäuresequenz gegenüber CFD und proCFD an zumindest einer Position eine andere Aminosäure auf. Nach einer noch weiteren Ausführungsform ist die erfindungsgemäße isoCFD gegenüber CFD um zumindest eine Aminosäure verlängert und/oder gegenüber proCFD um zumindest eine Aminosäure verkürzt, am C-Terminus und/oder vorzugsweise am N-Terminus.

Erfindungsgemäß sind unter Isoformen von CFD (isoCFD) jedoch keine mutierten Formen von CFD zu verstehen, wie bspw. die von Sprong et al. (a.a.O.) beschriebenen punktmutierten CFD-Formen. Erfindungsgemäß werden unter isoCFD auch keine CFD-Formen verstanden, die Polymorphismen aufweisen, wie bspw. die in der US 2003/0092620 beschriebenen Single-Nucleotid-Polymorphismen (SNPs).

Die Aminosäuresequenz der reifen bzw. aktiven Form des humanen CFD ist in dem beiliegenden Sequenzprotokoll unter SEQ ID Nr. 1 dargestellt. Die unreife Form von humanem CFD (proCFD) ist in dem beiliegenden Sequenzprotokoll unter SEQ ID Nr. 2 dargestellt.

Reifes humanes CFD weist einen theoretischen isoelektrischen Punkt von 6,85 und unreifes CFD (proCFD) von 7,99 auf. Die von den Erfindern ermittelten Isoformen von CFD (isoCFD) weisen theoretische isoelektrische Punkte von < 7,99 und > 6,85 auf. Die theoretischen isoelektrischen Punkte lassen sich nach der Methode berechnen, die beschrieben ist in Bjellqvist (1993), The focusing positions of polypeptides in immobilized pH gradients can be predicted from the amino acid sequences, electrophoresis, Vol. 14(10), Seiten 1023 bis 1031.

Aufgrund dieser Unterschiede lässt sich eine "isoCFD" mittels gängiger molekularbiologischer Methoden einfach und zuverlässig von CFD und proCFD unterscheiden, wie der isoelektrischen Fokussierung, einer Auftrennung nach dem Molekulargewicht oder der Masse, beispielsweise mittels SDS-Gelelektrophorese oder Massenspektrometrie.

"Zumindest eine isoCFD" bedeutet erfindungsgemäß, dass in der biologischen Probe auch zumindest zwei, zumindest drei, zumindest vier oder mehr unterschiedliche isoCFD enthalten sein können. Die Untersuchung in Schritt (2) kann sich deshalb nicht nur auf das Vorhandensein einer isoCFD sondern von mehreren unterschiedlichen isoCFD bzw. eines Musters von unterschiedlichen isoCFD richten.

Erfindungsgemäß wird unter einem "Lebewesen" ein jegliches Lebewesen verstanden, wobei das erfindungsgemäße Verfahren bevorzugt an einem Säugetier und weiter bevorzugt an einem Menschen durchführbar ist.

Erfindungsgemäß wird unter einer "biologischen Probe" eine jede potenziell CFD enthaltende Probe verstanden, wie eine Blut-, Urin-, Gelenkflüssigkeits- oder Sputumprobe oder aber auch eine Gewebeprobe, beispielsweise aus dem Fettgewebe.

Die Erfinder haben erstmals erkannt, dass isoCFD als diagnostischer Marker für eine Krankheit oder ein Risiko für die Entwicklung einer Krankheit verwendet werden kann. isoCFD eignet sich deshalb auch als Marker in einer therapiebegleitenden Diagnostik ("companion diagnostics").

Stellen einer Diagnose" umfasst die Feststellung, dass eine Krankheit oder ein Risiko für eine Krankheit bzw. Erkrankung vorliegt (positive Diagnose) oder nicht vorliegt (negative Diagnose). Es umfasst aber auch eine Stratifizierung des Lebewesens, also das Zuordnen eines Schweregrades der Erkrankung oder eines Risikogrades für eine Erkrankung oder das Fortschreitens der Erkrankung.

Vor diesem Hintergrund betrifft die Offenbarung auch eine Isoform des Komplementfaktors D (isoCFD) a|s diagnostischen Marker für eine Krankheit oder ein Risiko für die Entwicklung einer Krankheit.

Diese Ausgestaltung der Erfindung betrifft auch ein Muster von isoCFD, das in einer biologischen Probe eines Lebewesens nachweisbar ist, als diagnostischen Marker für eine Krankheit oder ein Risiko für die Entwicklung einer Krankheit.

Erfindungsgemäß wird hierbei unter einem "Muster" von isoCFD eine für das Lebewesen charakteristische Zusammensetzung verschiedener Isoformen von CFD bzw. ein charakteristisches Set von isoCFD verstanden. Diese unterschiedlichen Isoformen lassen sich bspw. in einer ein- oder zweidimensionalen Gelelektrophorese oder mittels Massenspektrometrie von einander trennen. Über diese Verfahren lassen sich den unterschiedlichen isoCFD unterschiedliche Messsignale zuordnen. Man erhält ein charakteristisches Signalmuster. Wenn sich dieses Muster von dem aus einem gesunden Referenzlebewesen unterscheidet, kann eine positive Diagnose getroffen werden.

Die Merkmale, Eigenschaften des erfindungsgemäßen Diagnoseverfahrens nebst dessen bevorzugten Weiterbildungen gelten für die erfindungsgemäße Isoform des Komplementfaktors D gleichermaßen.

Die der Erfindung zugrunde liegende Aufgabe wird hiermit vollkommen gelöst.

Nach einer bevorzugten Weiterbildung handelt es sich bei dem erfindungsgemäßen Verfahren um ein solches zur Diagnose einer Krankheit, die durch den alternativen Weg des Komplementsystems vermittelt wird, bzw. eines Risikos für die Entwicklung einer solchen Krankheit.

Diese Maßnahme hat den Vorteil, dass erstmals ein zuverlässiges und hochsensitives Verfahren bereitgestellt wird, mit dem Patienten hinsichtlich ihres Risikos, an einer durch den alternativen Weg des Komplementsystems stratifiziert werden können. Derartige Krankheiten sind im Stand der Technik hinreichend beschrieben, beispielsweise in der Publikation von Holers (2008; a.a.O.) oder in der Datenbank "MalaCards" des Weizmann Institute of Science. Vorzugsweise handelt es sich hierbei um Krankheiten, die ausgewählt sind aus der Gruppe bestehend aus Adipositas, Simpson-Golabi-Behmel-Syndrom (SGBS), zerebrale autosomal-rezessive Arteriopathie mit subkortikalen Infarkten und Leukenzephalopathie (CARASIL), Diabetes mellitus Typ 2, Faktor-D-Defizienz, atypisches hämolytisch-urämisches Syndrom (aHUS), paroxysmale nachtliche Hamoglobinurie (PNH), altersbedingte Makuladegeneration (AMD), rheumatoide Arthritis (RA), systemischer Lupus erythematodes (SLE), IgA-Nephropathie, Purpura Schönlein-Henoch, idiopathische membranoproliferative Glomerulonephritis, Post-Streptokokken-Krankheit, Myokarditis, multiple Sklerose (MS), Schädel-Hirn-Trauma, traumatische Rückenmarksverletzung, intestinaler und renaler Reperfusionsschaden, Antiphospholipid-Syndrom (APS), habituelles Abortsyndrom, Präeklampsie, Asthma, ANCA-assoziierte-pauci-immun-retinale Vasculitis, antikörper-vermittelte Hautkrankheit, Meningococcale Infektion, Anorexia Nervosa, Rhinitis, Hodgkins Lymphom, Non-Hodkins Lymphom, Neuronitis, Liposarcom, Sepsis, Urämie, Endothelitis, koronare Herzerkrankung, Multiples Myelom, Hiv-1, Intrakranielles Aneurysma, Rheumatoide Arthritis, McCune-Albright-Syndrom, Peritonitis, Hepatitis, Cerebritis, aortisches Aneurysma, corneale Fleck-Dystrophy, Herzinfarkt, dilative Kardiomyopathie, fibröse Dysplasie, LAMM-Syndrom ("deafness with labyrinthine aplasia microtia and microdontia"), intrakranielle Embolie.

Nach einer Variante des Verfahrens wird in Schritt (1) die biologische Probe auf das Vorhandensein zumindest einer solchen isoCFD untersucht, die im Vergleich zu CFD an ihrem N-Terminus um 1 bis 4, d.h. um 1 (Isoform "-4") oder um 2 (Isoform "-3") oder um 3 (Isoform "-2") oder um 4 Aminosäuren (Isoform "-1") verlängert ist.

Nach einer weiteren Variante wird in Schritt (1) die biologische Probe auf das Vorhandensein zumindest einer solchen isoCFD untersucht, die im Vergleich zu proCFD an ihrem N-Terminus um 1 bis 4, d.h. um 1 (Isoform "-1") oder um 2 (Isoform "-2") oder um 3 (Isoform "-3") oder um 4 Aminosäuren verkürzt ist (Isoform "-4").

Wie die Erfinder feststellen konnten, lassen sich derartige Isoformen von CFD in einer biologischen Probe bzw. Körperflüssigkeiten wie bspw. Blut oder Urin von Individuen finden, die entweder an einer der genannten Krankheiten erkrankt sind oder ein Risiko für eine Erkrankung aufweisen.

Nach einer bevorzugten Weiterbildung wird in Schritt (1) die biologische Probe auf das Vorhandensein einer isoCFD untersucht, die im Vergleich zu CFD an ihrem N-Terminus um eine Aminosäuresequenz verlängert ist, die ausgewählt ist aus der Gruppe bestehend aus: Arginin (R), Glyzin-Arginin (GR), Arginin-Glyzin-Arginin (RGR) und Prolin-Arginin-Glyzin-Arginin (PRGR), und/oder im Vergleich zu proCFD an ihrem N-Terminus um einer Aminosäuresequenz verkürzt ist, die ausgewählt ist aus der Gruppe bestehend aus: Prolin (P), Prolin-Prolin (PP), Prolin-Prolin-Arginin (PPR), Prolin-Prolin-Arginin-Glyzin und (PPRG).

Diese Maßnahme hat den Vorteil, dass spezifisch nach solchen Isoformen gesucht wird, die nach Erkenntnissen der Erfinder geeignete Indikatoren bzw. Biomarker darstellen.

Nach einer bevorzugten Weiterbildung des erfindungsgemäßen Verfahrens weist die isoCFD eine Aminosäuresequenz auf, die ausgewählt ist aus der Gruppe bestehend aus: SEQ ID Nr. 3, SEQ ID Nr. 4, SEQ ID Nr. 5, SEQ ID Nr. 6 und SEQ ID Nr. 7.

Diese Maßnahme hat den Vorteil, dass durch die Bereitstellung der Aminosäuresequenz der jeweiligen Isoformen von CFD eine noch zuverlässige Identifizierung der Biomarker ermöglicht wird.

Nach einer bevorzugten Weiterbildung des erfindungsgemäßen Verfahrens ist die biologische Probe ausgewählt aus der Gruppe bestehend aus: biologische Flüssigkeit, insbesondere Blutserum und Urin, oder Fettgewebe.

Diese Maßnahme hat den Vorteil, dass ein solches Ausgangsmaterial für die Durchführung des erfindungsgemäßen Verfahrens bereitgestellt wird, in dem sich die Isoformen von CFD besonders gut nachweisen lassen.

Nach einer bevorzugten Weiterbildung des erfindungsgemäßen Verfahrens erfolgt in Schritt (2) eine positive Diagnose, wenn die Konzentration von isoCFD in der biologischen Flüssigkeit ≥ 0,01 µg/ml, vorzugsweise ≥ 0,1 µg/ml, weiter vorzugsweise ≥ 1 µg/ml, weiter vorzugsweise ≥ 10 µg/ml, und weiter vorzugsweise ≥ 100 µg/ml ist. Eine negative Diagnose wird gestellt, wenn die Konzentration von isoCFD in der biologischen Flüssigkeit ≤ 0,01 µg/ml, vorzugsweise ≤ 0,1 µg/ml, weiter vorzugsweise ≤ 1 µg/ml, weiter vorzugsweise ≤ 10 µg/ml, und weiter vorzugsweise ≤ 100 µg/ml beträgt.

Diese Maßnahme hat den Vorteil, dass anhand von Schwellenwerten eine noch genauere Diagnose möglich ist. Die angegebenen Konzentrationen beziehen sich dabei auf eine einzelne Isoform von CFD. Nach einer alternativen Ausgestaltung beziehen sich die Konzentrationsangaben auf die Population sämtlicher Isoformen von CFD.

Nach einer bevorzugten Weiterbildung des erfindungsgemäßen Verfahrens erfolgt in Schritt (1) die Untersuchung der biologischen Probe mittels Massenspektrometrie und/oder eindimensionaler Gelelektrophorese und/oder zweidimensionaler Gelelektrophorese (2-DE), wobei weiter vorzugsweise in der eindimensionalen Gelelektrophorese eine isoelektrische Fokussierung (IEF) der Probe erfolgt, und/oder in einer Dimension der 2-DE eine isoelektrische Fokussierung (IEF) der Probe erfolgt, und/oder in einer bzw. der weiteren Dimension eine Auftrennung der Probe nach dem Molekulargewicht erfolgt.

Diese Maßnahme hat den Vorteil, dass molekularbiologische Methoden zum Einsatz kommen, mit denen auf besonders einfache Art und zuverlässige Art und Weise der Nachweis von isoCFD gelingt.

Die eindimensionale Gelelektrophorese nebst IEF hat gegenüber der 2-DE den Vorteil, dass sie weniger fehleranfällig, besser reproduzierbar und schneller durchführbar ist. Die Analysedauer wird von 3 Tagen auf unter 24 Stunden verkürzt und bietet die Möglichkeit, 40 Proben/Gel reproduzierbar parallel zu analysieren.

Die massenspektrometrische Analyse bietet hingegen den Vorteil, dass sie gänzlich ohne Antikörpereinsatz auskommt.

Nach einer bevorzugten Weiterbildung des erfindungsgemäßen Verfahrens erfolgt im Anschluss an die eindimensionale Gelelektrophorese und/oder die zweidimensionale Gelelektrophorese eine Westernblot-Analyse der biologischen Probe.

Im Rahmen diese Maßnahme lassen sich die auf einer Trägermembran übertragenen isoCFD zuverlässig und hochsensitiv mittels spezifischer Antikörper nachweisen. Diese analytische Methode ist für das erfindungsgemäße Verfahren besonders gut geeignet.

Ein weiterer Gegenstand der vorliegenden Erfindung betrifft ein Nucleinsäuremolekül, das für eine Isoform des Komplementfaktors D (isoCFD) codiert, die im Vergleich zu CFD an ihrem N-Terminus um 1 bis 4 Aminosäuren verlängert ist, und/oder im Vergleich zu proCFD an ihrem N-Terminus um 1 bis 4 Aminosäuren verkürzt ist.

Mittels eines solchen Nukleinsäuremoleküls kann in einem geeigneten Expressionssystem isoCFD hergestellt werden. Bei dem Nukleinsäuremolekül kann es sich um DNA, RNA, cDNA etc. handeln. Umfasst sind ferner Vektoren und Plasmide, die diejeweiligen Kodierungssequenzen enthalten. Das erfindungsgemäße Nukleinsäuremolekül kann vorzugsweise für ein isoCFD codieren, das eine Aminosäuresequenz aufweist, die ausgewählt ist aus der Gruppe bestehend aus: SEQ ID Nr. 3, SEQ ID Nr. 4, SEQ ID Nr. 5, SEQ ID Nr. 6 und SEQ ID Nr. 7.

Ein weiterer Gegenstand der Offenbarung betrifft ein Verfahren zum Auffinden von Wirksubstanzen, das folgende Schritte aufweist:
(1) Bereitstellen einer biologischen Probe, die isoCFD in einer Konzentration [Kₚᵣₑ] und/oder Aktivität [Aₚᵣₑ] enthält,
(2) Inkubation der biologischen Probe mit einer Testsubstanz unter physiologischen Bedingungen,
(3) Messen der Konzentration von isoCFD [Kₚₒₛₜ] und/oder der Aktivität von isoCFD [Aₚₒₛₜ],
(4) Identifizieren der Testsubstanz
   - als Wirksubstanz, wenn [Kₚₒₛₜ] ≠ [Kₚᵣₑ] und/oder [Aₚₒₛₜ] ≠ [Aₚᵣₑ], oder
   - als keine Wirksubstanz, wenn [Kₚₒₛₜ] = [Kₚᵣₑ] und [Aₚₒₛₜ] = [Aₚᵣₑ].

Mittels eines solchen Screeningverfahrens lassen sich erstmals auf sehr direkte Art und Weise Wirkstoffe auffinden, mit denen gezielt Krankheiten behandelt oder diesen vorgebeugt werden kann, die durch den alternativen Weg des Komplementsystems bzw. den Komplementfaktor D vermittelt werden. Eine Testsubstanz qualifiziert sich als Wirksubstanz, wenn sich durch die Inkubation der biologischen Probe mit der Testsubstanz bspw. die Konzentration von isoCFD und/oder deren Aktivität ändert, vorzugsweise erniedrigt. Dabei ist die [Kₚᵣₑ] und/oder [Aₚᵣₑ] bekannt.

Das Verfahren ist damit auch als Hochdurchsatzverfahren geeignet, beispielsweise zum Screening von Substanzbibliotheken.

Unter "physiologischen Bedingungen" werden solche Bedingungen verstanden, die im Organismus vorherrschen, beispielsweise eine Temperatur von 37 °C und einem pH-Wert von 7. Derartige Bedingungen reflektieren die Wirkung der Testsubstanz und die Interaktion der Komponenten des Komplementsystems, insbesondere des alternativen Wegs, im Körper eines zu behandelnden Individuums.

Unter der "Aktivität von isoCFD" wird die enzymati-sche oder katalytische Aktivität der Isoform von CFD in der biologischen Probe bzw. dem zugehörigen Organismus verstanden, die sich mittels dem Fachmann bekannter Test-systeme messen lässt.

Unter der "Konzentration von isoCFD" wird die Menge von isoCFD in der biologischen Probe bzw. dem zugehörigen Organismus pro Volumen-einheit verstanden.

Ein weiterer Gegenstand der vorliegenden Offenbarung betrifft ein Verfah-ren zum Auffinden einer Wirksubstanz, das folgende Schritte aufweist:
(1) Bereitstellen einer biologischen Probe,
(2) Bestimmung der in der biologischen Probe enthaltenden Isoformen des Komplementfaktors D (isoCFD) zum Erhalt eines ersten Musters Mₚᵣₑ,
(2) Inkubation der biologischen Probe mit einer Testsubstanz unter physiologischen Bedingungen,
(3) Bestimmung der in der biologischen Probe enthaltenden isoCFD zum Erhalt eines zweiten Musters Mₚₒₛₜ,
(4) Identifizieren der Testsubstanz
   - als Wirksubstanz, wenn Mₚₒₛₜ ≠ Mₒᵣₑ, oder
   - als keine Wirksubstanz, wenn Mₚₒₛₜ = Mₚᵣₑ.

Diese Variante des Screeningverfahrens bedient sich eines sogenannten Mustervergleichs, um Wirksubstanzen aufzufinden.

Unter einem "Muster" von isoCFD wird hierbei eine für die untersuchte Probe charakteristische Zusammensetzung verschiedener Isoformen von CFD bzw. ein charakteristisches Set von isoCFD verstanden. Diese unterschiedlichen Isoformen lassen sich bspw. in einer zweidimensionalen Gelelektrophorese oder mittels Massenspektrometrie von einander trennen. Uber diese Verfahren lassen sich den unterschiedlichen isoCFD unterschiedliche Messsignale zuordnen. Man erhält ein charak-teristisches Signalmuster. Wenn sich nun das vor der Inkubation der biologischen Probe erhaltene erste Muster Mₚᵣₑ von dem nach der Inkubation der biologischen Probe erhalte-ne zweite Muster Mₚₒₛₜ unterscheidet, erfolgt eine Identifizierung der Testsubstanz als Wirksubstanz. Zeigt die Testsubstanz keine Wirkung auf das Muster, scheidet die Testsubstanz als Wirksubstanz aus.

Die Merkmale, Eigenschaften und bevorzugten Ausgestaltungen des erfindungsgemäßen Diagnoseverfahrens gelten für die beiden Scree-ningverfahren entsprechend und umgekehrt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die vorliegende Erfindung wird nun anhand von Ausführungsbeispielen näher erläutert, aus denen sich weitere Merkmale, Eigenschaften und Vorteile der Erfindung ergeben. Die Ausführungsbeispiele haben dabei rein illustrativen Charakter und schränken die Reichweite der vorliegenden Erfindung nicht ein.

Dabei wird Bezug genommen auf die beiliegenden Figuren, in denen Folgendes dargestellt ist:

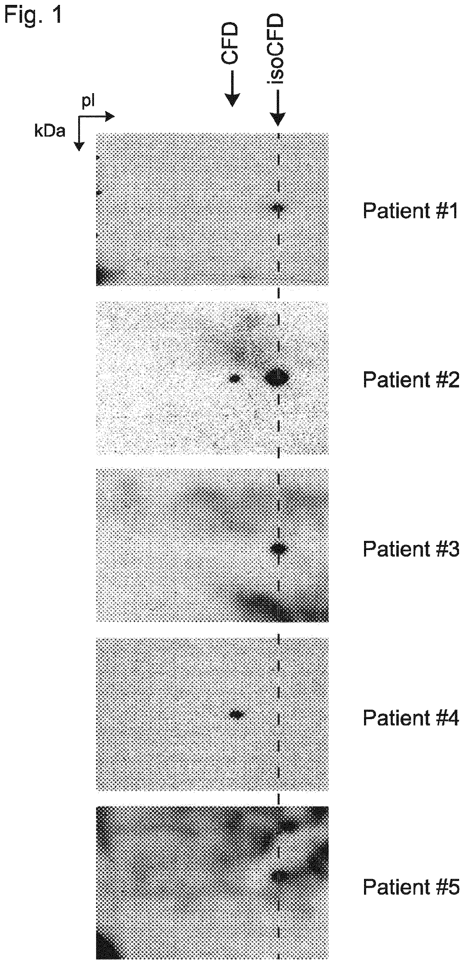

- Fig. 1: spezifische Detektion von CFD-Isoformen im Serum von fünf verschiedenen Adipositas-Patienten unter Verwendung einer zweidimensionalen Western blot-Analyse;

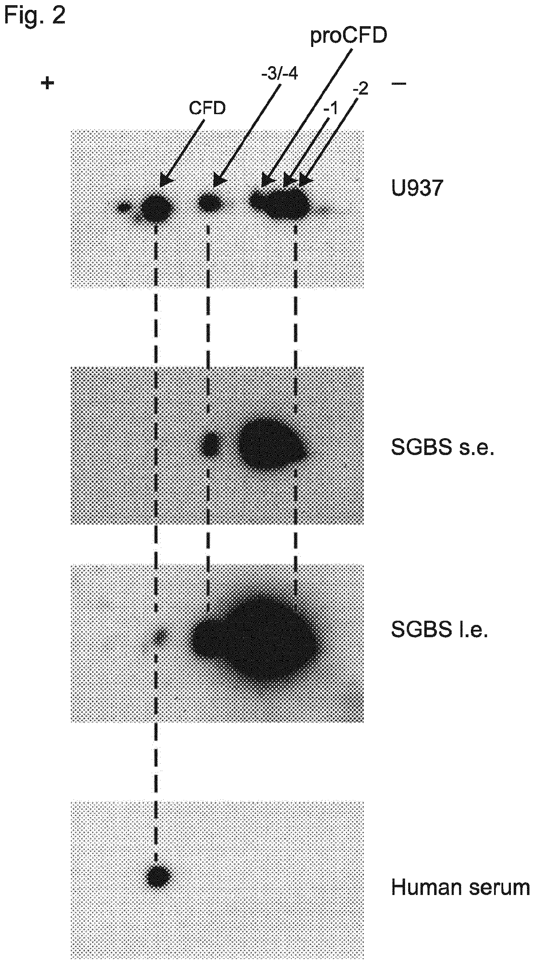

- Fig. 2: spezifische Detektion von CFD-Isoformen in einer humanen Monozytenzelllinie (U937) und einer Fettzelllinie aus Patienten mit Simpson-Golabi-Behmel-Syndrom (SGBS) im Vergleich zu humanem Serum einer gesunden Referenzperson.

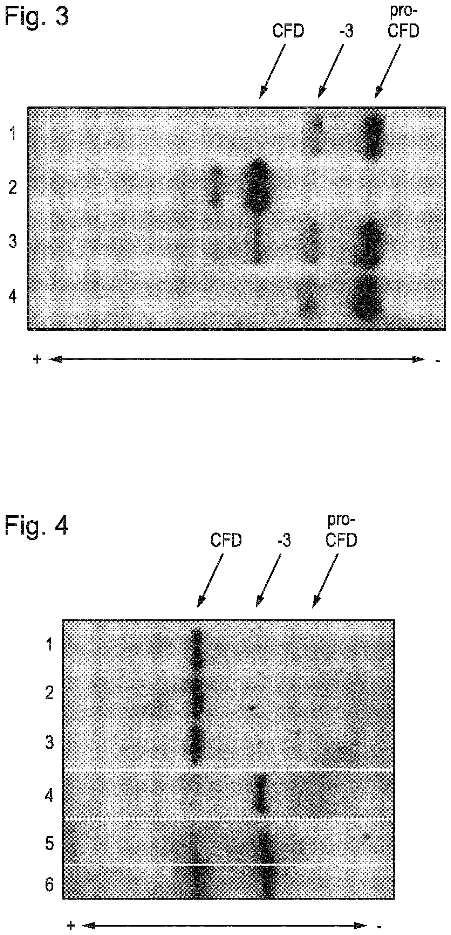

- Fig. 3: Aktueller Nachweis von CFD-Isoformen. Isoelektrische Fokussierung von 4 CFD-haltigen Proben in der eindimensionalen Gelelektrophorese, die mit unterschiedlichen Proteasen behandelt wurden. Eine dieser Proteasen ist in der Lage, proCFD vollständig zum aktiven Enzym (CFD) zu konvertieren (Spur 2), während eine andere zu einer Teilaktivierung führt (Spur 3). Es sind in allen Spuren weitere Isoformen nachweisbar.
- Fig. 4: Nachweis von CFD-Isoformen in klinischem Material. IEF-Immunoblot nach eindimensionaler Gelelektrophorese von 1-3) Serum, 4) retinalem Pigmentepithel, 5) visceralem Fett, und 6) subkutanem Fett. Im Gegensatz zum Serum (Spuren 1-3) findet in gesundem Pigmentepithel nahezu keine Prozessierung zum aktiven CFD statt (Spur 4), während es in den Fettgewebsproben zur Teilaktivierung kommt (Spuren 5-6).

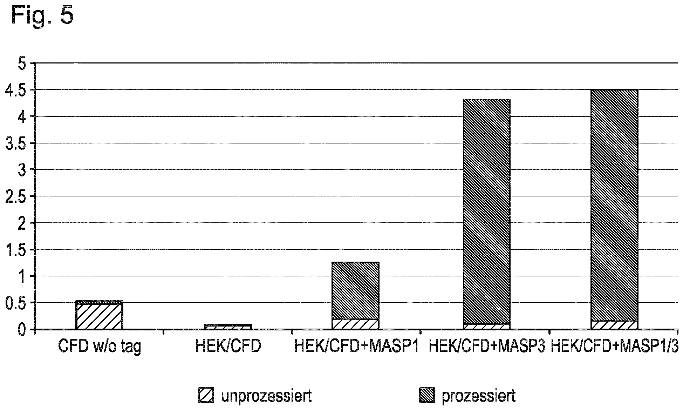

- Fig. 5: Nachweis des prozessierten N-Terminus von Profaktor D mittels zielgerichteter Massenspektrometrie (Summe der y7 Ionen ILGGREAE). proCFD-enthaltende Zellkulturüberstände (HEK/CFD) wurden mit verschiedenen rekombinanten Proteasen inkubiert, für die massenspektrometrische Proteinanalytik vorbereitet und schließlich zielgerichtete Peptide i) für das Coreprotein (2 Peptide), ii) für den prozessierten N-Terminus, und iii) für den unprozessierten N-Terminus in der QExactive Orbitrap vermessen. Nach Normalisierung auf die Signalintensitäten der Peptide aus dem Coreprotein, wurde der Grad der Prozessierung relativ quantifiziert. Nur die Gegenwart der Protease MASP3 zeigte in diesem Testsystem eine erfolgreiche Aktivierung von proCFD.

### Ausführungsbeispiele

### 1. Material und Methoden

### Patientenproben

Serumproben aus fünf krankhaft fettleibigen Patienten wurden von Professor Matthias Blüher, Universitätskrankenhaus Leipzig, Deutschland, erhalten. Nüchternblutproben wurden gesammelt und das Serum wurde durch Zentrifugation bei 3000 g für 10 Minuten separiert und bei -80 °C für die weitere Analyse gelagert.

Serumproben von einem gesunden Probanden wurden im Department für Augenheilkunde der Universität Tübingen entnommen, durch Zentrifugation bei 3000xg für 10 Minuten separiert und bei -80°C für die weitere Analyse gelagert. Proben von retinalem Pigmentepithel (RPE) wurde aus der Augenklinik der Universität Tübingen erhalten, viszerales und subcutanes Fett aus der chirurgischen Klinik. Die Gewebeproben wurden homogenisiert und bei -80°C bis zur weiteren Analyse gelagert.

### Zelllinien

Die humane Monozytenzelllinie U937 (ATCC; Manassas, VA) wurde in RPMI1640-Medium, versetzt mit 10 % FBS und 100 U/ml Penicillin und 100 µg/ml Streptomycin, bei 37 °C in einem Inkubator mit 5 % CO₂ kultiviert, bis eine Zelldichte von 1 x 10⁶ Zellen/ml erhalten wurde. Die Zellen wurden dann mit PPS gewaschen und weiter für 72 Stunden in serumfreiem Medium inkubiert. Konditioniertes Medium wurde gesammelt und unter Verwendung von Spin-Konzentratoren mit einem Ausschlussvolumen von 3 kDa konzentriert.

Die humane Präadipozytenzelllinie aus einem Patienten mit Simpson-Golabi-Behmel-Syndrom (SGBS) wurde von Professor Martin Babitsch, Universität Ulm, Deutschland, erhalten. Die Zellen wurden innerhalb von 14 Tagen in Adipozyten differenziert und in serumfreiem Medium kultiviert, wie die U937-Zellen.

### Behandeltes rekombinantes proCFD

Rekombinant in HEK293 Zellen hergestelltes humanes pro-CFD (ungereinigter Zellkulturüberstand) wurde nach Zusatz von diversen rekombinanten Proteasen (MASP1, MASP3, C1R und C1S) bei 37°C über Nacht inkubiert.

### Eindimensionale Gelelektrophorese/isoelektrische Fokussierung

Fertiggele des Typs IEFGel 6-11 40S (EDC, Tübingen) wurden in einer Multiphor II-Kammer (GE Healthcare) so positioniert, dass die Taschen des Gels in Richtung Anode zeigen. 10 µL Probe wurde jeweils in die Geltaschen luftblasenfrei pipettiert. Die isoelektrische Fokussierung (IEF) wurde wie folgt durchgeführt: 500 V für 20 Minuten, 1500 V für 90 Minuten, 1900 V für 20 Minuten.

### Zweidimensionale Gelelektrophorese (2-DE) /isoelektrische Fokussierung

Immobiline DryStrips, mit einem linearen pH-Gradienten von 3 bis 10, 18 cm, (GE Healthcare) wurden für 20 Stunden rehydriert, und zwar in einem Puffer enthaltend 8 M Harnstoff, 2 % CHAPS, 65 mM DTT % (v/v), 1 % Pharmalyte IPG Puffer 3-10, und Bromphenolblau (Trace). 200 µg von Serumproteinen wurden geladen und im Gel rehydriert. Für die Zellkulturüberstände wurde 1 ml serumfreies konditioniertes Medium unter Verwendung von Methanol/Chloroform präzipitiert, in Rehydrierungspuffer resuspendiert und auf gleiche Art und Weise geladen.

Die isoelektrische Fokussierung (IEF) wurde wie folgt durchgeführt: 500 V für 1 Stunde, 1000 V für 1 Stunde, 8000 V für 3 Stunden, und final 10.000 V für 4 Stunden. Die fokussierten IPG-Streifen wurden für 15 Minuten in 4 ml Äquilibrierungslösung äquilibriert, enthaltend 6 M Harnstoff, 75 mM Tris-Hcl-Puffer pH 6,8, 2 % (w/v) SDS, 29,3 % (v/v), 30 % Glycerin enthaltend 65 mM DTT. Das DTT wurde durch 260 mM lodoacetamid in der Äqulibrierungslösung ersetzt und es erfolgte eine Äquilibrierung für weitere 15 Minuten.

Ein Streifen von ca. 7 cm enthaltend sämtliche CFD-Isoformen wurden aus dem IPG-Streifen mit 18 cm ausgeschnitten und der Elektrophorese in der zweiten Dimension unter Verwendung von 10 %igen SDS-PAGE-Gelen mit 10 cm (Bio-Rad) zugeführt.

### Nachweis von CFD, proCFD und isoCFD

Zum Transferieren der Proteine auf eine Blotmembran wurde das Kontaktblotverfahren verwendet. Die PVDF-Membran wurde zunächst in 100 % Methanol aktiviert und dann dreimal 10 min in 1 × TBS-Puffer gespült. Das Gel wurde nach der isoelektrischen Fokussierung kurz gereinigt und auf eine Glasplatte mit dem Support nach unten platziert. Die Blotmembran wurde dann direkt auf das Gel gelegt und eventuelle Luftblasen herausgerollt. Fünf trockene Whatman-Filterpapiere, eine zweite Glasplatte und ein 1-2 kg Gewicht wurden darauf gestapelt, und nach 45 min konnte die Membran für die anschließende immunologische Detektion verwendet werden. Die aufgetrennten CFD-Isoformen wurden unter Verwendung eines Ziegen-Antikörpers gegen humanes CFD als primären Antikörper (AF1824; R & D Systems, Verdünnung 1:500) detektiert.

### Massenspektrometrie

Die massenspektrometrischen Untersuchungen erfolgten unter Anwendung von Standardprotokollen im Massenspektrometer QExactive Orbitrap.

### 2. Ergebnisse

In der Fig. 1 ist das Ergebnis einer zweidimensionalen gelelektrophoretischen Auftrennung und Westernblotanalyse von Blutseren aus fünf Patienten mit krankhafter Fettleibigkeit dargestellt. In den Patienten 1, 2, 3 und 5 sind Isoformen (isoCFD) von CFD festzustellen (gestrichelte Linie). Links davon findet man in den Patienten 2 und 4 die reife bzw. aktive Form von CFD (CFD). Die Isoformen von CFD stellen nach Erkenntnissen der Erfinder möglicherweise unvollständig prozessierte Varianten von pro-CFD dar.

In der Fig. 2 ist das Ergebnis einer gelelektrophoretischen zweidimensionalen Auftrennung und Analyse mittels Westernblot von Überständen aus humanen Monozyten (U937) und Adipozyten aus einem Patienten mit Simpson-Golabi-Behmel-Syndrom (SGBS) im Vergleich zu humanem Serum aus einer gesunden Referenzperson dargestellt. Die beiden Abbildungen betreffend SGBS unterscheiden sich in der Dauer der Exposition des Röntgenfilms. Einmal wurde kurz exponiert (s.e.), einmal zur besseren Visualisierung lang exponiert (I.e.). Sämtliche Abbildungen zeigen jeweils ein typisches Muster von isoCFD, die sich je nach untersuchter Probe voneinander unterscheiden. Dabei zeigt sich, dass im Blutserum des gesunden Spenders nur eine Form von CFD nachweisbar ist. Diese weist einen isoelektrischen Punkt von etwas weniger als 7,0 auf. In den U937 und SGBS-Zellen lassen sich Isoformen von CFD detektieren, die als "-4", "-3", "-2" und "-1" bezeichnet werden.

In einem weiteren Versuch wurde Blutserum aus Patienten mit zerebraler autosomal-rezessiver Arteriopathie mit subkortikalen Infarkten und Leukenzephalopathie (CARASIL) gelelektrophoretisch über zwei Dimensionen aufgetrennt. In der anschließenden Westernblotanalyse konnten ebenfalls mehrere Isoformen von CFD detektiert werden, die in Blutserum einer gesunden Referenzperson nicht feststellbar waren (Daten nicht gezeigt).

In der Fig. 3 ist das Ergebnis einer eindimensionalen Gelelektrophorese mit isoelektrischen Fokussierung und Westernblotanalyse von mit verschiedenen Proteasen behandeltem proCFD dargestellt. Es lassen sich verschiedene prominente Formen erkennen (Pfeile), unter anderem die isoCFD "-3". Mit MASP1 behandeltes pro-CFD wurde nicht stark prozessiert (Spur 1), während mit MASP3 behandeltes proCFD fast vollständig in aktives CFD überführt wurde, und sogar noch weiter prozessierte Formen (Spur 2) aufweist. Durch Behandlung mit C1R kam es zu einer partiellen Prozessierung / Aktivierung (Spur 3), während die Konvertase C1S kaum Effekte (Spur 4, wie MASP1) zeigt. Nach einer Behandlung mit C1R und C1S ist ebenfalls die isoCFD "-3" erkennbar.

In der Fig. 4 ist das Ergebnis einer isoelektrischen Fokussierung nach eindimensionaler Gelelektrophorese und Westernblotanalyse von einem Blutserum und verschiedenen Gewebelysaten, nämlich solchen aus retinalem Pigmentepithel (RPE) und viszeralem und subkutanem Fett, dargestellt. Im Blutserum (Spuren 1-3, Replikate) ist ausschließlich prozessiertes reifes CFD detektierbar (Pfeil mit "CFD"). Sowohl in den Extrakten von RPE (Spur 4) als auch in den beiden Fettgewebsproben (Spuren 5 und 6) ist die teilprozessierte isoCFD "-3" (Pfeil mit "-3") die prominenteste, während weitere Isoformen zu erkennen sind. Auffällig ist, dass die völlig unprozessierte Form des inaktiven Proenzyms ("proCFD") kaum detektierbar ist.

Um eine komplett andere Detektionsmethode zu etablieren, die unabhängig vom Nachweis mit Antikörpern ist, wurde eine zielgerichtete massenspektrometrische Analytik für den prozessierten N-Terminus von Profaktor D zu entwickelt. Nachdem eine Methode mit Hilfe von synthetischen Peptiden, die sowohl Teile des "Cores" von CFD als auch die zwei Hauptformen des N-Terminus abdeckten, auf dem QExactive Orbitrap Massenspektrometer aufgesetzt wurde, wurde rekombinantes proCFD in HEK-Zellen überexprimiert und im Überstand mittels limitierter Proteolyse und anschließender Peptidtrennung und -detektion nachgewiesen. Diese Überstände wurden dazu noch mit unterschiedlichen Proteasen vorinkubiert, um eine mögliche Konvertierung von proCFD hervorzurufen.

Die Ergebnisse der massenspektrometischen Untersuchung sind in Fig. 5 dargestellt: proCFD-enthaltende Zellkulturüberstände (HEK/CFD) wurden mit verschiedenen rekombinanten Proteasen inkubiert, für die massenspektrometrische Proteinanalytik vorbereitet und schließlich zielgerichtete Peptide i) für das Coreprotein (2 Peptide), ii) für den prozessierten N-Terminus, und iii) für den unprozessierten N-Terminus in der QExactive Orbitrap vermessen. Nach Normalisierung auf die Signalintensitäten der Peptide aus dem Coreprotein, wurde der Grad der Prozessierung relativ quantifiziert. Nur die Proteasen aus der MASP-Familie, i.e.L. MASP3 zeigten in diesem Testsystem eine erfolgreiche Aktivierung von proCFD.

Bei dem beschriebenen Experiment handelt es sich faktisch um einen Konvertase-Aktivitätstest, dem großes Potential in Entwicklung und Diagnostik zugemessen wird.

Nach Erkenntnissen der Erfinder lassen sich den aufgefundenen Isoformen spezifische Aminosäuresequenzen zuweisen, wie nachfolgend tabellarisch dargestellt:

**Tab. 1. Aufgefundene CFD-Varianten bzw. CFD-Isoformen in U937 una SGBS-Zellen; proCFD= unreife Form von CFD; reif = reife Form des CFD (CFD); "-1", "-2", "-3", "-4" = Isoformen von CFD; die kursiv dargestellten Aminosäuren lassen sich in der der proCFD finden, die nicht-kursiv dargestellten Aminosäuren begrenzen das reife CFD.**

| CFD-Variante bzw. -Isoform | N-terminale Sequenz | Theoretischer pl | Vollständige Sequenz |
|---|---|---|---|
| proCFD | *PPRGR*ILGGR ... SEQ ID Nr. 7 | 7,99 | SEQ ID Nr. 2 |
| -1 | *PRGR*ILGGR ... SEQ ID Nr. 8 | 7,99 | SEQ ID Nr. 3 |
| -2 | *RGR*ILGGR ... SEQ ID Nr. 9 | 7,78 | SEQ ID Nr. 4 |
| -3 | *GRI*LGGR ... SEQ ID Nr. 10 | 7,26 | SEQ ID Nr. 5 |
| -4 | *R*ILGGR ... SEQ ID Nr. 11 | 7,26 | SEQ ID Nr. 6 |
| reif | ILGGR ... SEQ ID Nr. 12 | 6,85 | SEQ ID Nr. 1 |

Nachfolgend ist der N-Terminus von CFD bzw. proCFD und entsprechenden Isoformen dargestellt. Aminosäuren des reifen Proteins sind in großen Buchstaben in Normalschrift dargestellt, der Abschnitt, der sich in den Isoformen von CFD und proCFD finden lässt, ist in Kursivschrift dargestellt, und der Abschnitt, der das Signalpeptid betrifft, ist unterstrichen:

### MHSWERLAVLVLLGAAACAAPPRGRILGGR ... (SEQ ID Nr. 13)

Nach Erkenntnissen der Erfinder unterscheiden sich die aufgefundenen Isoformen von CFD gegenüber dem proCFD (SEQ ID Nr. 2) durch eine N-terminale Verkürzung des Peptids um eine Aminosäure (Prolin, "-1"; SEQ ID Nr. 3), zwei Aminosäuren (Prolin-Prolin, "-2"; SEQ ID Nr. 4), drei Aminosäuren (Prolin-Prolin-Arginin, "-3"; SEQ ID Nr. 5) bzw. vier Aminosäuren (Prolin-Prolin-Arginin-Glyzin, "-4"; SEQ ID Nr. 6) bzw. um entsprechend hinzugefügte Aminosäuren gegenüber dem reifen CFD (SEQ ID Nr. 1). Die Erfinder gehen davon aus, dass die Isoformen auf eine unvollständige Prozessierung von proCFD zurückgehen.

Die Erfinder konnten zeigen, dass der Nachweis von isoCFD nicht nur über klassische gelelektirsche Methoden mit isoelektrischer Fokussierung sondern auch über moderner massenspektrometrische Tests erfolgen kann. Damit gelingt auch der Nachweis der Konvertierung von proCFD in biologischen Proben.

### 3. Fazit

Die Erfinder haben erstmals erkannt, dass Isoformen von CFD geeignete diagnostische bzw. biologische Marker darstellen. Anhand einer biologischen Probe eines Lebewesens, wie einer Blut- oder Urinprobe, kann über das Vorhandensein oder die Abwesenheit dieser Marker eine Aussage über eine Erkrankung oder eine Veranlagung für eine Erkrankung getroffen werden, insbesondere einer durch den alternativen Weg des Komplementsystems vermittelten Krankheit. Ferner lassen sich die Isoformen von CFD nutzen, um neue Wirksubstanzen aufzufinden, mit denen durch den alternativen Weg des Komplementsystems, insbesondere durch den Komplementfaktor D, vermittelte Krankheiten behandelt oder vorgebeugt werden können.

### Sequenzen

SEQ ID Nr. 1: Aminosäuresequenz reifes humanes CFD (CFD)
SEQ ID Nr. 2: Aminosäuresequenz unreifes humanes CFD (proCFD)
SEQ ID Nr. 3: Aminosäuresequenz Isoform "-1" von humanem CFD (isoCFD "-1")
SEQ ID Nr. 4: Aminosäuresequenz Isoform "-2" von humanem CFD (isoCFD "-2")
SEQ ID Nr. 5: Aminosäuresequenz Isoform "-3" von humanem CFD (isoCFD "-3")
SEQ ID Nr. 6: Aminosäuresequenz Isoform "-4" von humanem CFD (isoCFD "-4")
SEQ ID Nr. 7: Aminosäuresequenz N-Terminus von proCFD
SEQ ID Nr. 8: Aminosäuresequenz N-Terminus von isoCFD "-1"
SEQ ID Nr. 9: Aminosäuresequenz N-Terminus von isoCFD "-2"
SEQ ID Nr. 10: Aminosäuresequenz N-Terminus von isoCFD "-3"
SEQ ID Nr. 11: Aminosäuresequenz N-Terminus von isoCFD "-4"
SEQ ID Nr. 12: Aminosäuresequenz N-Terminus von CFD
SEQ ID Nr. 13: Aminosäuresequenz N-Terminus von CFD/proCFD/Signalpeptid

## Patentansprüche

1. Ex-vivo-Verfahren zur Diagnose einer Krankheit oder eines Risikos für die Entwicklung einer Krankheit bei einem Lebewesen, das folgende Schritte aufweist:
(1) Untersuchung einer von einem Lebewesen bereitgestellten biologischen Probe auf das Vorhandensein von zumindest einer Isoform des Komplementfaktors D (CFD), die im Vergleich zu CFD an ihrem N-Terminus um 1 bis 4 Aminosäuren verlängert ist, und/oder im Vergleich zu proCFD an ihrem N-Terminus um 1 bis 4 Aminosäuren verkürzt ist (isoCFD),
(2) Stellen einer positiven Diagnose bei einem positiven Nachweis von zumindest einer isoCFD in der biologischen Probe,
oder
Stellen einer negativen Diagnose bei einem negativen Nachweis von zumindest einer isoCFD in der biologischen Probe.

2. Verfahren nach Anspruch 1, **dadurch gekennzeichnet, dass** die Krankheit durch den alternativen Weg des Komplementsystems vermittelt wird, und vorzugsweise ausgewählt ist aus der Gruppe bestehend aus: Adipositas, Simpson-Golabi-Behmel-Syndrom (SGBS), zerebrale autosomal-rezessive Arteriopathie mit subkortikalen Infarkten und Leukenzephalopathie (CARASIL), Diabetes mellitus Typ 2, Faktor-D-Defizienz, atypisches hämolytisch-urämisches Syndrom (aHUS), paroxysmale nächtliche Hämoglobinurie (PNH), altersbedingte Makuladegeneration (AMD), rheumatoide Arthritis (RA), systemischer Lupus erythematodes (SLE), IgA-Nephropathie, Purpura Schönlein-Henoch, idiopathische membranoproliferative Glomerulonephritis, Post-Streptokokken-Krankheit, Myokarditis, multiple Sklerose (MS), Schädel-Hirn-Trauma, traumatische Rückenmarksverletzung, intestinaler und renaler Reperfusionsschaden, Antiphospholipid-Syndrom (APS), habituelles Abortsyndrom, Präeklampsie, Asthma, ANCA-assoziierte-pauci-immun-retinale Vasculitis, antikörper-vermittelte Hautkrankheit.

3. Verfahren nach einem der vorherigen Ansprüche, **dadurch gekennzeichnet, dass** in Schritt (1) die biologische Probe auf das Vorhandensein einer isoCFD untersucht wird, die
im Vergleich zu CFD an ihrem N-Terminus um eine Aminosäuresequenz verlängert ist, die ausgewählt ist aus der Gruppe bestehend aus: Arginin (R), Glyzin-Arginin (GR), Arginin-Glyzin-Arginin (RGR) und Prolin-Arginin-Glyzin-Arginin (PRGR), und/oder
im Vergleich zu proCFD an ihrem N-Terminus um eine Aminosäuresequenz verkürzt ist, die ausgewählt ist aus der Gruppe bestehend aus: Prolin (P), Prolin-Prolin (PP), Prolin-Prolin-Arginin (PPR), Prolin-Prolin-Arginin-Glyzin und (PPRG), und
die vorzugsweise
eine Aminosäuresequenz aufweist, die ausgewählt ist aus der Gruppe bestehend aus: SEQ ID Nr. 3, SEQ ID Nr. 4, SEQ ID Nr. 5 und SEQ ID Nr. 6.

4. Verfahren nach einem der vorherigen Ansprüche, **dadurch gekennzeichnet, dass** die biologische Probe ausgewählt ist aus der Gruppe bestehend aus: biologische Flüssigkeit, insbesondere Blutserum, Urin; Gelenkflüssigkeit, Sputum, und Fettgewebe.

5. Verfahren nach Anspruch 4, **dadurch gekennzeichnet, dass** in Schritt (2)
- eine positive Diagnose gestellt wird, wenn die Konzentration von isoCFD in der biologischen Flüssigkeit ≥ 0,01 µg/ml, vorzugsweise ≥ 0,1 µg/ml, weiter vorzugsweise ≥ 1 µg/ml, weiter vorzugsweise ≥ 10 µg/ml, und weiter vorzugsweise ≥ 100 µg/ml ist, und
- eine negative Diagnose gestellt wird, wenn die Konzentration von isoCFD in der biologischen Flüssigkeit ≤ 0,01 µg/ml, vorzugsweise ≤ 0,1 µg/ml, weiter vorzugsweise ≤ 1 µg/ml, weiter vorzugsweise ≤ 10 µg/ml, und weiter vorzugsweise ≤ 100 µg/ml ist.

6. Verfahren nach einem der vorherigen Ansprüche, **dadurch gekennzeichnet, dass** in Schritt (1) die Untersuchung der biologischen Probe mittels Massenspektrometrie und/oder eindimensionaler Gelelektrophorese oder zweidimensionaler Gelelektrophorese (2-DE) erfolgt.

7. Verfahren nach Anspruch 6, **dadurch gekennzeichnet, dass** in der eindimensionalen Gelelektrophorese eine isoelektrische Fokussierung der Probe erfolgt, und/oder in der 2-DE in einer Dimension eine isoelektrische Fokussierung der Probe erfolgt, und/oder dass in der 2-DE in einer Dimension eine Auftrennung der Probe nach dem Molekulargewicht erfolgt.

8. Verfahren nach Anspruch 6 oder 7, **dadurch gekennzeichnet, dass** im Anschluss an die eindimensionalen Gelelektrophorese und/oder die 2-DE eine Westernblot-Analyse der biologischen Probe erfolgt.

9. Verwendung einer Isoform des Komplementfaktors D (CFD), die im Vergleich zu CFD an ihrem N-Terminus um 1 bis 4 Aminosäuren verlängert ist, und/oder im Vergleich zu proCFD an ihrem N-Terminus um 1 bis 4 Aminosäuren verkürzt ist (isoCFD) als diagnostischer Marker für eine Krankheit oder ein Risiko für die Entwicklung einer Krankheit, und
vorzugsweise einer durch den alternativen Weg des Komplementsystems vermittelten Krankheit,
weiter vorzugsweise ausgewählt aus der Gruppe bestehend aus: Adipositas, Simpson-Golabi-Behmel-Syndrom (SGBS), zerebrale autosomal-rezessive Arteriopathie mit subkortikalen Infarkten und Leukenzephalopathie (CARASIL), Diabetes mellitus Typ 2, Faktor-D-Defizienz, atypisches hämolytisch-urämisches Syndrom (aHUS), paroxysmale nächtliche Hämoglobinurie (PNH), altersbedingte Makuladegeneration (AMD), rheumatoide Arthritis (RA), systemischer Lupus erythematodes (SLE), IgA-Nephropathie, Purpura Schönlein-Henoch, idiopathische membranoproliferative Glomerulonephritis, Post-Streptokokken-Krankheit, Myokarditis, multiple Sklerose (MS), Schädel-Hirn-Trauma, traumatische Rückenmarksverletzung, intestinaler und renaler Reperfusionsschaden, Antiphospholipid-Syndrom (APS), habituelles Abortsyndrom, Präeklampsie, Asthma, ANCA-assoziierte-pauci-immun-retinale Vasculitis, antikörper-vermittelte Hautkrankheit Meningococcale Infektion, Anorexia Nervosa, Rhinitis, Hodgkins Lymphom, Non-Hodgkins Lymphom, Neuronitis, Liposarcom, Sepsis, Urämie, Endothelitis, koronare Herzerkrankung, Multiples Myelom, Hiv-1, Intrakranielles Aneurysma, McCune-Albright-Syndrom, Peritonitis, Hepatitis, Cerebritis, aortisches Aneurysma, corneale Fleck-Dystrophy, Herzinfarkt, dilative Kardiomyopathie, fibröse Dysplasie, LAMM-Syndrom ("deafness with labyrinthine aplasia microtia and microdontia"), intrakranielle Embolie.

10. Verwendung nach Anspruch 9,
die
im Vergleich zu CFD an ihrem N-Terminus um eine Aminosäuresequenz verlängert ist, die ausgewählt ist aus der Gruppe bestehend aus: Arginin (R), Glyzin-Arginin (GR), Arginin-Glyzin-Arginin (RGR) und Prolin-Arginin-Glyzin-Arginin (PRGR), und/oder
im Vergleich zu proCFD an ihrem N-Terminus um eine Aminosäuresequenz verkürzt ist, die ausgewählt ist aus der Gruppe bestehend aus: Prolin (P), Prolin-Prolin (PP), Prolin-Prolin-Arginin (PPR), Prolin-Prolin-Arginin-Glyzin und (PPRG), und
vorzugsweise
eine Aminosäuresequenz aufweist, die ausgewählt ist aus der Gruppe bestehend aus: SEQ ID Nr. 3, SEQ ID Nr. 4, SEQ ID Nr. 5 und SEQ ID Nr. 6.

11. Verwendung nach einem der vorherigen Ansprüche als diagnostischer Marker in einer biologischen Probe, vorzugsweise ausgewählt aus der Gruppe bestehend aus: biologische Flüssigkeit, insbesondere Blutserum, Urin; Fettgewebe.

12. Verwendung nach Anspruch 11, **dadurch gekennzeichnet, dass**
- eine positive Diagnose gestellt wird, wenn die Konzentration von isoCFD im Blutserum und/oder Urin ≥ 0,01 µg/ml, vorzugsweise ≥ 0,1 µg/ml, weiter vorzugsweise ≥ 1 µg/ml, weiter vorzugsweise ≥ 10 µg/ml, und weiter vorzugsweise ≥ 100 µg/ml ist, und
- eine negative Diagnose gestellt wird, wenn die Konzentration von isoCFD im Blutserum und/oder Urin ≤ 0,01 µg/ml, vorzugsweise ≤ 0,1 µg/ml, weiter vorzugsweise ≤ 1 µg/ml, weiter vorzugsweise ≤ 10 µg/ml, und weiter vorzugsweise ≤ 100 µg/ml ist.

13. Nucleinsäuremolekül, das für eine Isoform des Komplementfaktors D, die im Vergleich zu CFD an ihrem N-Terminus um 1 bis 4 Aminosäuren verlängert ist, und/oder im Vergleich zu proCFD an ihrem N-Terminus um 1 bis 4 Aminosäuren verkürzt ist, (isoCFD) codiert.

## Claims

1. An ex vivo method for the diagnosis of a disease or a risk for the development of a disease in a living being, comprising the following steps:
(1) Examination of a biological sample provided from a living being for the presence of at least one isoform of the complement factor D (isoCFD), which in comparison with CFD is extended at its N terminus by 1 to 4 amino acids, and/or in comparison with proCFD is shortened at its N terminus by 1 to 4 amino acids (isoCFD),
(2) Making a positive diagnosis in case of a positive detection of at least one isoCFD in the biological sample,
or
making a negative diagnosis in case of a negative detection of at least one isoCFD in the biological sample.

2. The method of claim 1, **characterized in that** the disease is mediated via the alternative pathway of the complement system, and
is preferably selected from the group consisting of: obesity, Simpson-Golabi-Behmel syndrome (SGBS), cerebral autosomal dominant arteriopathy with subcortical infarcts and leukoencephalopathy (CARASIL), diabetes mellitus type 2, factor D deficiency, atypical haemolytic uraemic syndrome (aHUS), paroxysmal nocturnal hemoglobinuria (PNH), age related macula degeneration (AMD), rheumatoid arthritis (RA), systemic lupus erythematosus (SLE), IgA nephropathy, Henoch-Schonlein purpura, idiopathic membranoproliferative glomerulonephritis,
post streptococcal disease, myocarditis, multiple sclerosis (MS), traumatic brain injury, traumatic spinal cord injury, intestinal and renal reperfusion injury, antiphospholipid syndrome (APS), habitual abort syndrome, pre-eclampsia, asthma, ANCA associated pauci immune retinal vasculitis, antibody mediated skin disease.

3. The method of any of the preceding claims, **characterized in that** in step (1) the biological sample is examined for the presence of an isoCFD which
in comparison with CFD is extended at its N terminus by an amino acid sequence which is selected from the group consisting of: arginine (R), glycine-arginine (GR), arginine-glycine-arginine (RGR) and proline-arginine-glycine-arginine (PRGR), and/or
in comparison with proCFD is shortened at its N terminus by an amino acid sequence which is selected from the group consisting of: proline (P), proline-proline (PP), proline-proline-arginine (PPR), proline-proline-arginine-glycine (PPRG), and
which preferably
comprises an amino acid sequence which is selected from the group consisting of: SEQ ID No. 3, SEQ ID No. 4, SEQ ID No. 5, and SEQ ID No. 6.

4. The method according to any of the preceding claims, **characterized in that** the biological sample is selected from the group consisting of: biological liquid, in particular blood serum, urine; joint fluid, sputum, and fatty tissue.

5. The method of claim 4, **characterized in that** in step (2)
- a positive diagnosis is made if the concentration of isoCFD in the biological liquid is ≥ 0.01 µg/ml, preferably ≥ 0.1 µg/ml, further preferably ≥ 1 µg/ml, further preferably ≥ 10 µg/ml, and further preferably ≥ 100 µg/ml, and
- a negative diagnosis is made if the concentration of isoCFD in the biological sample is ≤ 0.01 µg/ml, preferably ≤ 0.1 µg/ml, further preferably ≤ 1 µg/ml, further preferably ≤ 10 µg/ml, and further preferably ≤ 100 µg/ml.

6. The method of any of the preceding claims, **characterized in that** in step (1) the examination of the biological sample is carried out by means of mass spectrometry and/or one-dimensional gel electrophoresis or two-dimensional gel electrophoresis (2-DE).

7. The method of claim 6, **characterized in that** in the one-dimensional gel electrophoresis an isoelectric focusing of the sample is carried out, and/or in the 2-DE in one dimension an isoelectric focusing of the sample is carried out, and/or in the 2-DE in one dimension a separation of the sample according to the molecular weight is carried out.

8. The method of claim 6 or 7, **characterized in that** after the one-dimensional gel electrophoresis and/or the 2-DE a Westernblot analysis of the biological sample is carried out.

9. A use of an isoform of the complement factor D (CFD) which in comparison with CFD is extended at its N terminus by 1 to 4 amino acids, and/or in comparison with proCFD is shortened at its N terminus by 1 to 4 amino acids (isoCFD) as a diagnostic marker for a disease or a risk for the development of a disease, and
preferably a disease mediated via the alternative pathway of the complement system,
further preferably selected from the group consisting of: obesity, Simpson-Golabi-Behmel syndrome (SGBS), cerebral autosomal dominant arteriopathy with subcortical infarcts and leukoencephalopathy (CARASIL), diabetes mellitus type 2, factor D deficiency, atypical haemolytic uraemic syndrome (aHUS), paroxysmal nocturnal hemoglobinuria (PNH), age related macula degeneration (AMD), rheumatoid arthritis (RA), systemic lupus erythematosus (SLE), IgA nephropathy, Henoch-Schonlein purpura, idiopathic membranoproliferative glomerulonephritis, post streptococcal disease, myocarditis, multiple sclerosis (MS), traumatic brain injury, traumatic spinal cord injury, intestinal and renal reperfusion injury, antiphospholipid syndrome (APS), habitual abort syndrome, pre-eclampsia, asthma, ANCA associated pauci immune retinal vasculitis, antibody mediated skin disease, meningococcal infection, anorexia nervosa, rhinitis, Hodgkin's lymphoma, non-Hodgkin's lymphoma, neuronitis, liposarcoma, sepsis, uremia, endotheliitis, coronary heart disease, multiple myeloma, Hiv 1, intracranial aneurysm, rheumatoid arthritis, McCune Albright syndrome, peritonitis, hepatitis, cerebritis, aortic aneurysm, Fleck corneal dystrophy, heart attack, dilated cardiomyopathy, fibrous dysplasia, LAMM syndrome ("deafness with labirithine aplasia microtia and microdontia"), intracranial embolism.

10. The use of claim 9, which
in comparison with CFD is extended at its N terminus by an amino acid sequence selected from the group consisting of: arginine (R), glycine-arginine (GR), arginine-glycine-arginine (RGR) and proline-arginine-glycine-arginine (PRGR), and/or
in comparison with proCFD is shortened at its N terminus by an amino acid sequence which is selected from the group consisting of: proline (P), proline-proline (PP), proline-proline-arginine (PPR), proline-proline-arginine-glycine (PPRG), and
which preferably
comprises an amino acid sequence which is selected from the group consisting of: SEQ ID No. 3, SEQ ID No. 4, SEQ ID No. 5, and SEQ ID No. 6.

11. The use of any of the preceding claims as a diagnostic marker in a biological sample, preferably selected from the group consisting of: biological liquid, in particular blood serum, urine; fatty tissue.

12. The use of claim 11, **characterized in that**
- a positive diagnosis is made if the concentration of isoCFD in the biological liquid is ≥ 0.01 µg/ml, preferably ≥ 0.1 µg/ml, further preferably ≥ 1 µg/ml, further preferably ≥ 10 µg/ml, and further preferably ≥ 100 µg/ml, and
- a negative diagnosis is made if the concentration of isoCFD in the biological sample is ≤ 0.01 µg/ml, preferably ≤ 0.1 µg/ml, further preferably ≤ 1 µg/ml, further preferably ≤ 10 µg/ml, and further preferably ≤ 100 µg/ml.

13. A nucleic acid molecule encoding an isoform of the complement factor D which in comparison with CFD is extended at its N terminus by 1 to 4 amino acids, and/or in comparison with proCFD is shortened at its N terminus by 1 to 4 amino acids (isoCFD).

## Revendications

1. Procédé ex-vivo de diagnostic d'une maladie ou d'un risque de développement d'une maladie pour un organisme vivant, qui présente des étapes suivantes :
(1) l'étude de la présence, sur un échantillon biologique fourni par un organisme vivant, d'au moins une isoforme du facteur du complément D (CFD), qui est prolongée, en comparaison avec le CFD, sur son terminus N, de 1 à 4 acides aminés et/ou est raccourcie, en comparaison avec le proCFD, sur son terminus N, de 1 à 4 acides aminés (isoCFD),
(2) la pose d'un diagnostic positif en cas de détection confirmée d'au moins une isoCFD dans l'échantillon biologique
ou
la pose d'un diagnostic négatif en cas d'absence confirmée d'au moins une isoCFD dans l'échantillon biologique.

2. Procédé selon la revendication 1, **caractérisé en ce que** la maladie est médiée par la voie alterne du système du complément, et
est choisie de préférence parmi le groupe constitué : de l'obésité, du syndrome de Simpson-Golabi-Behmel (SGBS), de l'artériopathie cérébrale autosomique récessive avec infarctus sous-corticaux et leucoencéphalopathie (CARASIL), du diabète sucré de type 2, du déficit en facteur D, du syndrome hémolytique et urémique atypique (SHUa), de l'hémoglobinurie paroxystique nocturne (HPN), de la dégénérescence maculaire liée à l'âge (DMLA), de la polyarthrite rhumatoïde (PR), du lupus érythémateux systémique (LES), de la néphropathie à IgA, du purpura de Henoch-Schönlein, de la glomérulonéphrite membranoproliférative idiopathique, du syndrome post-streptococcique, d'une myocardite, de la sclérose multiple (SM), d'un traumatisme crânio-cérébral, d'une lésion médullaire traumatique, d'une lésion de reperfusion intestinale et rénale, du syndrome des anti-phospholipides (SAPL), du syndrome d'avortement à répétition, de la prééclampsie, de l'asthme, de la vascularite rétinienne pauci-immune associée aux ANCA, de la dermatose médiée par des anticorps.

3. Procédé selon l'une quelconque des revendications précédentes, **caractérisé en ce que** lors de l'étape (1)
est étudiée la présence d'une isoCFD sur l'échantillon biologique, qui est prolongée, en comparaison avec le CFD, sur son terminus N, d'une séquence d'acides aminés qui est choisie parmi le groupe constitué de : l'arginine (R), la glycine-arginine (GR), l'arginine-glycine-arginine (RGR) et la proline-arginine-glycine-arginine (PRGR), et/ou
est raccourcie, en comparaison avec le proCFD, sur son terminus N, d'une séquence d'acides aminés qui est choisie parmi le groupe constitué de : la proline (P), la proline-proline (PP), la proline-proline-arginine (PPR), la proline-proline-arginine-glycine (PPRG) et
qui présente de préférence
une séquence d'acides aminés qui est choisie parmi le groupe constitué de : SEQ ID n° 3, SEQ ID n° 4, SED ID n° 5 et SEQ ID n° 6.

4. Procédé selon l'une quelconque des revendications précédentes, **caractérisé en ce que** l'échantillon biologique est choisi parmi le groupe constitué : de liquide biologique, en particulier de sérum sanguin, d'urine ; de liquide synovial, d'expectoration et de tissu adipeux.

5. Procédé selon la revendication 4, **caractérisé en ce que** lors de l'étape (2),
- un diagnostic positif est posé quand la concentration de isoCFD dans le liquide biologique est ≥ 0,01 µg/ml, de préférence ≥ 0,1 µg/ml, de manière davantage préférée ≥ 1 µg/ml, de manière davantage préférée ≥ 10 µg/ml et de manière davantage préférée ≥ 100 µg/ml, et
- un diagnostic négatif est posé quand la concentration de isoCFD dans le liquide biologique est ≤ 0,01 µg/ml, de préférence ≤ 0,1 µg/ml, de manière davantage préférée ≤ 1 µg/ml, de manière davantage préférée ≤ 10 µg/ml et de manière davantage préférée ≤ 100 µg/ml.

6. Procédé selon l'une quelconque des revendications précédentes, **caractérisé en ce que** lors de l'étape (1),
l'étude de l'échantillon biologique est effectuée au moyen d'une spectrométrie de masse et/ou d'une électrophorèse monodimensionnelle sur gel ou d'une électrophorèse bidimensionnelle sur gel (2-DE).

7. Procédé selon la revendication 6, **caractérisé en ce que** dans l'électrophorèse monodimensionnelle sur gel, une concentration isoélectrique de l'échantillon est effectuée, et/ou dans la 2-DE, une focalisation isoélectrique de l'échantillon est effectuée dans une dimension, et/ou que dans la 2-DE, une séparation de l'échantillon selon le poids moléculaire est effectuée dans une dimension.

8. Procédé selon la revendication 6 ou 7, **caractérisé en ce qu'**une analyse Western Blot de l'échantillon biologique est effectuée suite à l'électrophorèse monodimensionnelle sur gel et/ou à la 2-DE.

9. Utilisation d'une isoforme du facteur de complément D (CFD), qui est prolongée, en comparaison avec le CFD, sur son terminus N, de 1 à 4 acides aminés et/ou est raccourcie, en comparaison avec le proCFD, sur son terminus N, de 1 à 4 acides aminés (isoCFD) en tant que marqueur pour le diagnostic d'une maladie ou d'un risque de développement d'une maladie, et
de préférence d'une maladie médiée par la voie alterne du système du complément,
choisie par ailleurs de préférence parmi le groupe constitué : de l'obésité, du syndrome de Simpson-Golabi-Behmel (SGBS), de l'artériopathie cérébrale autosomique récessive avec infarctus sous-corticaux et leucoencéphalopathie (CARASIL), du diabète sucré de type 2, du déficit en facteur D, du syndrome hémolytique et urémique atypique (SHUa), de l'hémoglobinurie paroxystique nocturne (HPN), de la dégénérescence maculaire liée à l'âge (DMLA), de la polyarthrite rhumatoïde (PR), du lupus érythémateux systémique (LES), de la néphropathie à IgA, du purpura de Henoch-Schönlein, de la glomérulonéphrite membranoproliférative idiopathique, du syndrome post-streptococcique, d'une myocardite, de la sclérose multiple (SM), d'un traumatisme crânio-cérébral, d'une lésion médullaire traumatique, d'une lésion de reperfusion intestinale et rénale, du syndrome des anti-phospholipides (SAPL), du syndrome d'avortement à répétition, de la prééclampsie, de l'asthme, de la vascularite rétinienne pauci-immune associée aux ANCA, de la dermatose médiée par des anticorps, de la méningite à méningocoques, de l'anorexie mentale, d'une rhinite, d'un lymphome hodgkinien, d'un lymphome non hodgkinien, d'une neuronite, d'un liposarcome, d'un sepsis, d'une urémie, d'une endothélite, d'une maladie cardiaque coronarienne, du myélome multiple, du VIH-1, d'un anévrisme intracrânien, du syndrome de McCune Albright, d'une péritonite, d'une hépatite, d'une cérébrite, d'un anévrisme aortique, de la dystrophie cornéenne de Fleck, d'un infarctus du myocarde, d'une cardiomyopathie dilatée, de la dysplasie fibreuse, du syndrome LAMM (surdité avec aplasie labyrinthique, microtie et microdontie), d'une thrombose veineuse cérébrale.

10. Utilisation selon la revendication 9,
qui
est prolongée, en comparaison avec le CFD, sur son terminus N, d'une séquence d'acides aminés qui est choisie parmi le groupe constitué de : l'arginine (R), la glycine-arginine (GR), l'arginine-glycine-arginine (RGR) et la proline-arginine-glycine-arginine (PRGR), et/ou
est raccourcie, en comparaison avec le proCFD, sur son terminus N, d'une séquence d'acides aminés qui est choisie parmi le groupe constitué de : la proline (P), la proline-proline (PP), la proline-proline-arginine (PPR), la proline-proline-arginine-glycine (PPRG) et
qui présente de préférence
une séquence d'acides aminés qui est choisie parmi le groupe constitué de : SEQ ID n° 3, SEQ ID n° 4, SED ID n° 5 et SEQ ID n° 6.

11. Utilisation selon l'une quelconque des revendications précédentes en tant que marqueur pour le diagnostic sur un échantillon biologique de préférence choisi parmi le groupe constitué : de liquide biologique, en particulier de sérum sanguin, d'urine ; de tissus adipeux.

12. Utilisation selon la revendication 11, **caractérisée en ce que**
- un diagnostic positif est posé quand la concentration de isoCFD dans le sérum sanguin et/ou l'urine est ≥ 0,01 µg/ml, de préférence ≥ 0,1 µg/ml, de manière davantage préférée ≥ 1 µg/ml, de manière davantage préférée ≥ 10 µg/ml et de manière davantage préférée ≥ 100 µg/ml, et
- un diagnostic négatif est posé quand la concentration de isoCFD dans le sérum sanguin et/ou l'urine est ≤ 0,01 µg/ml, de préférence ≤ 0,1 µg/ml, de manière davantage préférée ≤ 1 µg/ml, de manière davantage préférée ≤ 10 µg/ml et de manière davantage préférée ≤ 100 µg/ml.

13. Molécule d'acide nucléique, qui code une isoforme du facteur du complément D, qui est prolongée, en comparaison avec le CFD, sur son terminus N, de 1 à 4 acides aminés et/ou est raccourcie, en comparaison avec proCFD, sur son terminus N, de 1 à 4 acides aminés (isoCFD).
